# EUROPEAN PATENT SPECIFICATION

(11) **EP 4 731 189 B1**
(45) Date of publication and mention of the grant of the patent: **19.08.2026**
(21) Application number: 24733959.1
(22) Date of filing: 19.06.2024
(51) Int. Cl.: A61K 9/16, A61K 31/439

(54) **SOLID ORAL FORMULATION OF PUDAFENSINE**
FESTE ORALE FORMULIERUNG VON PUDAFENSIN
FORMULATION ORALE SOLIDE DE PUDAFENSINE

(30) Priority: 20.06.2023 EP 23180312; 05.10.2023 EP 23201954
(43) Date of publication of application: 29.04.2026
(73) Proprietor: Initiator Pharma A/S, 2200 Copenhagen N (DK)
(72) Inventor: THOMSEN, Mikael, 2200 Copenhagen N (DK); EGEBRO, René, 2200 Copenhagen N (DK)
(74) Representative: Holm, Peter Joakim
(86) International application number: PCT/EP2024/067038
(87) International publication number: WO 2024/261026

(56) References cited:
- WO-A1-2011/092061
- SANDEEP KALEPU ET AL: "Insoluble drug delivery strategies: review of recent advances and business prospects", ACTA PHARMACEUTICA SINICA B, vol. 5, no. 5, 1 September 2015 (2015-09-01), pages 442 - 453, XP055433512, ISSN: 2211-3835, DOI: 10.1016/j.apsb.2015.07.003

## Description

### Technical field

The present disclosure relates to a composition comprising pellets comprising or consisting of: a) a pellet core; and b) a drug layer covering the core, the drug layer comprising a compound of formula (I) 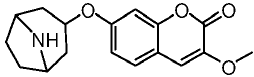 formula (I) or a pharmaceutically acceptable salt thereof.

### Background

Pudafensine (the compound Exo-7-[(-8-azabicyclo[3.2.1]octan-3-yl)oxy]-3-methoxy-chromen-2-one also known as IP2015) is and mono-amine re-uptake inhibitor under clinical development. In the nervous system, the compound increases the level of neurotransmitters, which has utility in treatment of central nervous system disorders.

Given the compound's effect on the central nervous system, it is necessary that oral formulations exhibit a robust release profile. In some applications, it is desirable to obtain a complete release of the compound as fast as possible, in order to achieve the therapeutic effects as soon as possible.

WO 2011/092061 A1 discloses monoamine reuptake inhibitors and proposes them for treatment of for treatment of CNS conditions. The compound of formula (I) is exemplified as a monoamine reuptake inhibitor.

Sandeep Kalepu et al. discloses methods for improving the solubility and bioavailability of insoluble drugs, such a commercially available composition of the insoluble drug itraconazole formulated by spraying an organic solution of itraconazole and HPMC onto a core of sugar beads.

Until now, no known solid formulations of the compound have shown to produce an effective release of the compound. The compound suffers from low solubility, which poses a challenge both in the manufacture of such formulations and for ensuring fast complete release upon oral administration of the compound.

Accordingly, there is a strong un-met need for solid forms that can ensure a consistent and fast release of pudafensine upon oral administration.

### Summary

The invention is defined by the claims. Any subject matter falling outside the scope of the claims is provided for information purposes only.

The inventors have surprisingly found that specific solid formulations of pudafensine can provide fast immediate release of the compound and high absorption in humans despite the drugs low solubility. The formulations according to the present disclosure are able to provide complete release of the compound in minutes, as demonstrated by the examples, and the drug is properly absorbed in humans and detected in plasma. This is highly advantageous in the clinical application of pudafensine since it allows a safe and robust administration of the compound with a fast release and on-set of the therapeutic effect.

In one aspect, the present disclosure provides for composition comprising pellets, said pellets comprising or consisting of,
a. a pellet core; and
b. a drug layer covering the core, the drug layer comprising a compound of formula (I) 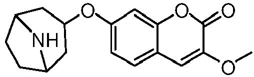 formula (I) or a pharmaceutically acceptable salt thereof.

In one aspect, the present disclosure provides for a unit dosage form comprising the composition described herein.

In a final aspect, the present disclosure provides for method of preparing a composition comprising pellets, the method comprising:
a) providing pellet core,
b) providing a drug layer solution comprising a compound of formula (I) 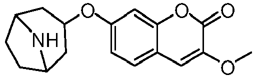 formula (I), or a pharmaceutically acceptable salt thereof; and
c) coating the drug layer solution on to the pellet core to form a drug layer.

### Description of Drawings

Figure 1: Dissolution rate of IR pellets and IR capsules having 10 mg or 5 mg of pudafensine. **A, B**: IR pellets 10 mg pudafensine; **C**: IR pellets 5 mg pudafensine; **D**: IR capsules 5 mg pudafensine. Further experimental details are given in Example 2.
Figure 2: Data from a Phase Ilb study with the compound IP2015 in ED patients show their response to question Q3 of the international index of erectile function scale 15 (IIEF-15): When you attempted intercourse, how often were you able to penetrate (enter) your partner during the last week?
Figure 3: Data from a Phase Ilb study with the compound IP2015 in ED patients show their response to question Q4 in the International Index of erectile function scale 15 (IIEF-15): During sexual intercourse, how often were you able to maintain your erection after you had penetrated (entered) your partner?
Figure 4: Data from a Phase Ilb study with the compound IP2015 in ED patients show their overall response to questions of the International Index of erectile function scale 15 (IIEF-15).

### Definitions

"Cₘₐₓ" is a term used in pharmacokinetics to refer to the maximum (or peak) serum concentration that a drug achieves in a specified compartment or test area of the body after the drug has been administrated and prior to the administration of a second dose.

"tₘₐₓ" is the term used in pharmacokinetics to describe the time at which the Cₘₐₓ is observed.

As used herein the terms "inert core" and "inert sphere" refer to a pharmaceutically acceptable core for use in pharmaceutical formulations which core is inert. For example, the "inert core" or "inert sphere" refers to a pharmaceutically acceptable pellet that does not contain a drug substance.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

By "pudafensine," "IP2015" or "compound I" is meant the compound of formula I. The compound of formula I is:

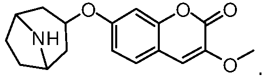

The term "pharmaceutically acceptable salt" of a compound refers to a salt that is pharmaceutically acceptable, as defined herein, and preferably possesses the desired pharmacological activity of the parent compound. Pharmaceutically acceptable salts include acid addition salts formed with inorganic acids; or formed with organic acids; or salts formed when an acidic proton present in the parent compound either is replaced by a metal ion; or coordinates with an organic or inorganic base.

As used herein, "formulation" is the result of combining different substances, including the active ingredient, to produce a final product.

As used herein, the term 'weight of the pellets' refers to the total weight of the pellets including the drug layer within the composition, e.g., the total weight of the components comprising the pellet core, drug layer, and any other layer on the pellet. In capsule formulations, this does not include the weight of the capsule shell.

### Detailed description

The present disclosure provides for composition comprising pellets, said pellets comprising or consisting of,
a) a pellet core; and
b) a drug layer covering the core, the drug layer comprising a compound of formula (I) 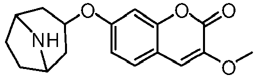 formula (I) or a pharmaceutically acceptable salt thereof.

### A composition

Pellets offer a high degree of flexibility in the design and development of oral dosage forms. Microcrystalline cellulose pellets (MCC) and sugars are well-known materials in pellet technology to use as core materials. Water-insolubleinert pellet cores are made of microcrystalline cellulose or silica, while water-soluble inert pellet cores are composed of sugar, such as sucrose, xylitol, mannitol, lactose; starch or salts. Both material classes show desirable characteristics, such as a narrow particle size distribution, sphericity, and surface smoothness and may be used according to the invention. A person of skill in the art will be able to determine suitable materials and suppliers of spheres suitable as core for pellets of the compositions according to the present disclosure.

In one embodiment, the pellets comprise an inert pellet core. In one embodiment, the pellet core comprises or consist of microcrystalline cellulose (MCC). MCC is a commercially available chemical (CAS 9004-34-6) and its pellets are commercially available in multiple size ranges, for example Cellet^{®} or Celphere^{™}.

The size of the pellet core may be any suitable size, for example at least 100 µm. Different size of pellet cores may be used, for example pellet cores with sizes from 100 µm to 1500 µm, such as from 100 µm to 500 µm, such as from 500 µm to 1000 µm or from 1000 µm to 1500 µm.

Methods to coat pellet compositions are well known in the art. They can both start from a solution, dispersion or powder that is coated onto the pellets layer by layer. Well-established methods to coat pellets are for example: a fluidized bed technology, by compression coating, würster coating, pan coating, centrifugal or rotor coating.

In one embodiment, the drug layer further comprises a binder. Binders are used to avoid the sticking of the drug on the pellets and provide a uniform coating on the pellets. The binder is mixed with the drug in a solution and then coated onto the inert pellets. It was surprisingly found that the presence of a binder, such as HPMC, in the coating solution allowed for homogeneous coating of pudafensine on to the inert pellet coat despite the compound's low solubility.

Known binders are for example sucrose, starch, hydroxypropyl methyl cellulose (HPMC) hydroxypropyl cellulose (HPC), gelatin or polyvinylpyrrolidone. Thus, in one embodiment, the binder is selected from: sucrose, starch, hydroxypropyl methyl cellulose (HPMC), hydroxypropyl cellulose (HPC), gelatin or polyvinylpyrrolidone (PVP); polyalkylene glycols or polyalkylene oxides, such as polyethylene oxide (PEO); or mixtures thereof.

In one embodiment, the drug layer comprises the binder in an amount from 0.5% to 10% by weight of the composition or by weight of the pellets, such as from 0.5% to 5%, such as from 1% to 5%, such as from 2% to 5%, such as 2%, 3%, 4% or 5% by weight of the composition or by weight of the pellets. Particularly, in one embodiment the drug layer comprises the binder, as described herein, in an amount from 0.5% to 10% by weight of the pellets, such as from 0.5% to 5%, such as 2%, 3%, 4% or 5% by weight of the pellets.

In one embodiment, the binder is hydroxypropyl methyl cellulose (HPMC). HPMC (CAS number 9004-65-3) is a cellulose derivative wherein some free hydroxyl groups in cellulose have been substituted with hydroxypropyl and methyl groups, different grades of HPMC having different multiple weight, degree hydroxypropyl or methyl groups and viscosities are commercially available and suitable for use in formulation development. In one embodiment, the drug layer comprises HPMC in an amount from about 0.5% to about 10% by weight of the composition or by weight of the pellets . In one embodiment, the drug layer comprises HPMC in an amount from about 1% to 5% by weight of the composition or by weight of the pellets, such as about 2%, 3%, 4% or 5% by weight of the composition or by weight of the pellets. Particularly, in one embodiment the drug layer comprises HPMC, as described herein, in an amount from 0.5% to 10% by weight of the pellets, such as from 0.5% to 5%, such as 2%, 3%, 4% or 5% by weight of the pellets.

In one embodiment, the drug layer comprises additional excipients such as pH modifiers and/or stabilizers. The inventors unexpectedly found that the addition of sodium sulphite (also known as sodium sulphite or Na₂SO₃, CAS number 7757-83-7) in the drug layer increased the dissolution rate of pudafensine from the drug layer. Thus, in one embodiment the drug layer further comprises sodium sulphite. The examples demonstrate that the presence of sodium sulphite in the drug layer accelerated the dissolution rate of pudafensine from the formulation.

The pH modifier, the stabilizer or the sodium sulphite may be present for example in an amount from about 0.05 % to about 1.5 % by weight of the composition or the pellets, such as for example from 0.05 % to 0.5 % by weight, such as from 0.5 % to 1.0% by weight, such as for example 1.0 % to 1.5 % by weight of the composition or by weight of the pellets. In one embodiment, the stabilizer or the sodium sulphite is present in an amount from about 0.05% to about 1.5% by weight of the pellets.

In one embodiment, the sodium sulphite is present in an amount of about 0.3 % to 0.8% by weight of the pellets. In one embodiment, the sodium sulphite is present in an amount of about 0.5% by weight of the pellets.

In one embodiment, the compound of formula I, or pharmaceutically acceptable salt thereof, is present in an amount from about 0.5% to about 5 % by weight of the total composition or by weight of the pellets. For example, the compound of formula I may be present in an amount from 0.5 % to 1 %, such as from 1 % to 2 %, such as from 2 % to 3 %, such as from 3 % to 4 %, such as from 4 % to 5 % by weight of the total composition or by weight of the pellets. In one embodiment, the compound of formula I may be present from 2.5% to 3.5% by weight of the composition or by weight of the pellets.

In one embodiment, the compound of formula I, or pharmaceutically acceptable salt thereof, is present in an amount from about 0.5% to about 5% by weight of the pellets. For example, the compound of formula I is present in an amount from 0.5 % to 1 %, such as from 1 % to 2 %, such as from 2 % to 3 %, such as from 3 % to 4 %, such as from 4 % to 5 % by weight of the pellets. In one embodiment, the compound of formula I is present in an amount from 2.5% to 3.5% by weight of the pellets.

In one embodiment, the compound of formula I is according to formula la 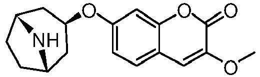 formula (la),
or a pharmaceutically acceptable salt thereof.

In one embodiment, the compound of formula (I) is Exo-7-[(-8-azabicyclo[3.2.1]octan-3-yl)oxy]-3-methoxy-chromen-2-one or a pharmaceutically acceptable salt thereof.

In one embodiment, the pharmaceutically acceptable salt of the compound of formula (I) or formula (la) is a hydrochloride salt.

In one embodiment, the composition is an immediate release (IR) composition of the compound of formula I.

Thus, in one embodiment the present disclosure provides for a composition which is an immediate release composition of an active ingredient consisting of a compound of formula I, 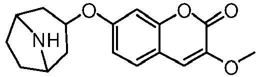 formula (I), or a pharmaceutically acceptable salt thereof, the composition comprising pellets, said pellets comprising or consisting of:
a) an inert pellet core comprising microcrystalline cellulose (MCC);
b) a drug layer comprising: the compound of formula (I) or a pharmaceutically acceptable salt thereof in an amount of about 0.5% to about 5% by weight of the total composition or by weight of the pellets and HPMC in an amount of 0.5% to about 5% by weight of the total composition or by weight of the pellets, which layer covers the inert core.

In one embodiment the present disclosure provides for a composition which is an immediate release composition of an active ingredient consisting of a compound of formula I, 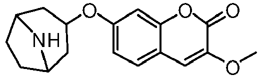 formula (I), or a pharmaceutically acceptable salt thereof, the composition comprising pellets, said pellets comprising or consisting of:
a) an inert pellet core comprising microcrystalline cellulose (MCC);
b) a drug layer comprising: the compound of formula (I), or a pharmaceutically acceptable salt thereof, in an amount of about 0.5% to about 5% by weight of the pellets and HPMC in an amount of 0.5% to about 5% by weight of the pellets, which layer covers the inert core.

In one embodiment, the drug layer further comprises other excipients such as pH modifiers or stabilizers, as described herein. In one embodiment, the drug layer further comprises sodium sulphite as described herein.

In one embodiment, the drug layer further comprises HPMC as described herein.

In one embodiment, the composition according to the present disclosure comprises an amount of the compound of formula I of about 0.5 mg to about 30 mg, such as from 1 mg to 20 mg, such as from 1 mg to about 16 mg, such as 1 mg to about 10 mg, such as from 2 mg to about 10 mg, such as from 3 mg to about 10 mg, such as from 4 mg to about 10 mg. For example, the composition may comprise 1, 2, 3, 4, 5, 6, 7, 8, 9, or 10 mg of the compound of formula I. In one embodiment, the composition comprises about 5 mg of the compound of formula I.

### Dosage form

The present disclosure provides for dosage form, such as a pharmaceutical dosage form, comprising the composition as described herein. In one embodiment, the dosage form is a unit dosage form. Methods to manufacturing dosage forms, including suitable excipients and materials will be known to the skilled artisan.

The unit dosage form or the pharmaceutical dosage form is in one embodiment a solid dosage form. In one embodiment, the unit dosage form or the pharmaceutical dosage form is a capsule.

Dosage forms are designed to facilitate the safe and effective delivery of active compounds to patients.

Thus, in one embodiment, the composition according to the present disclosure is in the form of a unit dosage form or a pharmaceutical dosage form. In one embodiment, the composition according to the present disclosure is a capsule.

In one embodiment, the composition is for oral administration.

### Immediate release

The inventors have surprisingly shown that the compositions according to the present disclosure provide for a fast and immediate release of pudafensine in the gastrointestinal tract after oral administration.

The examples demonstrate that the compositions according to the present disclosure provide for a fast release of pudafensine, with complete release upon 5 minutes in physiological conditions, allowing for a fast absorption of the drug despite its poor solubility.

In one embodiment, the composition provides a pharmacokinetic profile having a single mean average plasma concentration peak upon oral administration.

In one embodiment, the composition provides a Cₘₐₓ of the compound of formula I after oral administration of 80% to 125% of the Cₘₐₓ provided by an equivalent amount of the compound of formula I administered orally as an aqueous solution. In one embodiment, the composition provides for a Cₘₐₓ of the compound of formula I upon oral administration from 5.0 ng/mL to 13.0 ng/mL, such as from 6.0 ng/mL to 10.0 ng/mL, or 7.0 ng/mL to 9.5 ng/mL.

In one embodiment, the composition provides in a mean Cₘₐₓ of the compound of formula I from 6.0 ng/mL to 10.0 ng/mL, for example from 6.0 ng/mL to 7.0 ng/mL, or from 7.0 ng/mL to 8.0 ng/mL, or from 8.0 ng/mL to 9.0 ng/mL, or from 9.0 ng/mL to 10.0 ng/mL.

In one embodiment, the composition increases tₘₐₓ of the compound of formula I compared to an aqueous solution of the compound of formula I after oral administration by no more than 150 minutes.

In one embodiment, the composition provides a tₘₐₓ of the compound of formula I from about 2 to 8 hours, such as from 3 to 5 hours after oral administration, such as tₘₐₓ 3, 4, or 5 hours.

The compositions according to the present disclosure provide for an immediate release of the compound of formula I. An immediate release may be characterized by the dissolution rate of the active ingredient from the composition. A person of skill in the art is aware of methods to determine dissolution rate of solid dosage forms, such as according to the US Pharmacopeia, such as with a USP-I or a USP-II apparatus, with suitable methods of detection of the active ingredient such as HPLC or ELISA.

In one embodiment, the composition releases the compound of formula I at a rate of more than 90% released in the first hour as measured in a USP-I or a USP-II apparatus at pH 1.0 at 37 °C.

In one embodiment, the composition releases the compound of formula I at a rate of more than 90% release in the first 0.5 hours, such as more than 90% release in the first 10 minutes, such as more than 90% release in the first 5 minutes as measured in a USP-I or a USP-II apparatus at pH 1.0 at 37 °C.

In one embodiment, the composition releases the compound of formula I at a rate of essentially 100%, or complete release, in the first 5 minutes as measured in a USP-I or a USP-II apparatus at pH 1.0 at 37 °C. Surprisingly, it was found that addition of sodium sulphite in the drug layer produced an increase in the release that allowed for complete release after 5 min in dissolution rate tests simulating stomach pH.

The exposure of a substance may be measured as the "area under the curve" or "AUC" of the compound in the bloodstream. As it is well-known in the art, this may be calculated by plotting the concentration of the drug in the bloodstream against time and determining the integral of the resulting curve, also known as the "area under the curve" of "AUC".

The amount of drug in the bloodstream may be detected at different time-points by well known methods in the art such as HPLC, LC/MS, ELISA, or other suitable analytical methods. Methods to calculate the integral of the plot are also well known to some one of skill in the art. The integral of the plot, or area under the curve, may be calculated within a specific time interval, such as within the first 24, 48, or 72 hours. In general, the term "AUC₀-ₜ" refers to the area under the curve for the first "t" hours, for example as in "AUC₀₋₂₄" referring to the area under the curve within the first 24 hours . The term "AUC₀-_{inf}" or "AUC_{inf}" refers to the area under the curves where the last point for integration has been extrapolated to infinity.

In one embodiment, the composition provides for a AUC₀₋₇₂ of the compound of formula I of at least 90% of the AUC₀₋₇₂ of an aqueous solution of an equivalent amount of the compound of formula I after oral administration.

In one embodiment, the composition provides for a AUC_{0-inf} of the compound of formula I of at least 90% of the AUC_{0-inf} of an aqueous solution of an equivalent amount of the compound of formula I after oral administration. In one embodiment, the composition provides for an AUC_{0-inf} of the compound of formula I upon oral administration from 150 to 550 h·ng/mL, such as AUC_{0-inf} from 180 to 480 h·ng/mL, or AUC_{0-inf} from 250 to 350 h·ng/mL, such as AUC_{0-inf} of about 300 h·ng/mL.

In one embodiment, the composition provides for mean AUC_{0-inf} of the compound of formula I upon oral administration from 200 h·ng/mL to 400 h·ng/mL, such as mean AUC_{0-inf} from 250 h·ng/mL to 350 h·ng/mL.

Thus, in one aspect the present disclosure provides for an immediate release composition of a compound of formula (I), said composition providing a pharmacokinetic profile upon administration characterized by having one or more of:
- a Cₘₐₓ of the compound of formula I from 5.0 ng/mL to 13.0 ng/mL, such as from 6.0 ng/mL to 10.0 ng/mL, such as from 7.5 ng/mL to 9.0 ng/mL,
- a tₘₐₓ of the compound of formula I from about 2 to 8 hours, such as from 3 to 5 hours,
- an AUC_{0-inf} of the compound of formula I from 180 to 480 h·ng/mL, such as AUC_{0-inf} from 250 to 350 h·ng/mL, such as AUC_{0-inf} of about 300 h·ng/mL.

In one embodiment, said immediate release composition comprises the compound of formula (I), a pellet core, a binder, pH modifiers or stabilizers as described herein in the section "A composition".

### Method of preparation

In a final aspect, the present disclosure provides for method of preparing a composition comprising pellets, the method comprising:
a) providing pellet core,
b) providing a drug layer solution comprising a compound of formula (I) 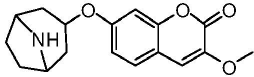 formula (I), or a pharmaceutically acceptable salt thereof; and
c) coating the drug layer solution on to the pellet core to form a drug layer.

In one embodiment of the method, the pellet core, or the drug layer, are as described herein in the section "Composition".

In one embodiment of the method, the drug layer solution comprises a binder or a stabilizer according as described herein in the section "Composition".

In one embodiment of the method, the drug layer solution comprises a solvent or dispersant, wherein said solvent or dispersant comprises or consist of an aqueous solution.

### Examples

### Example 1: Preparation of formulations

### Aim

To describe the preparation of compositions according to the present disclosure.

### Materials and Methods

**Materials.** Microcrystalline cellulose spheres (Celphere CP-507 particle size range 500-710 µm), sodium sulphite anhydrous, hydroxypropylmethyl cellulose (HPMC, Methocel E5 premium LV) and pudafensine monohydrate monohydrochloride salt.

Sodium sulphite, HPMC 5cps and pudafensine are mixed in purified water then stirred to get uniform dispersion. Then the solution is coated onto the inert pellet core (Celphere CP-507) in a fluidized bed (GPCG 1.1 bowl, Gun nozzle size: 1.2 mm, ADP size: type B). Finally, the drug-layered pellets are filled into Swedish Orange Capsule size 0.

For pudafensine solutions, a drug in bottle product is prepared as following: pudafensine is dissolved in water for injection containing 50 mg/mL of hydroxypropyl 2-beta cyclodextrin (HPβCD)
The drug substance pudafensine was used as a monohydrate and monohydrochloride salt. Hence the quantity per bottle, or capsule or administration is calculated based on the amount of free base.

### Results

IP2015 compositions where prepared according to the tables below.

**Table 1. Composition of IP2015 immediate release (IR) pellets**

| | | **IP2015 IR pellets** | | |
|---|---|---|---|---|
| **S. No** | **Purpose** | **Ingredients** | **Qty (g)** | **% w/w** |
| | | **Drug layering** | | |
| 1 | Core pellets | Celphere CP-507 | 1400.0 | 92.729 |
| 2 | Drug substance | IP2015^{a} | 48.37 | 3.204 |
| 3 | Binder | HPMC E5 (Methocel E5 premium LV) | 53.40 | 3.537 |
| 4 | pH modifier/alkalizer | Sodium sulphite anhydrous | 8.00 | 0.530 |
| 5 | Dispersant | Purified Water | q.s | q.s |
| | | **Total** | **1509.6** | **100.000** |

| | | | | |
|---|---|---|---|---|
| ^{a}The drug substance is a monohydrate and monohydrochloride salt. The quantity of free base may be calculated by analysis of anhydrous and solvent-free basis, by methods well known in the art. For the present batch 60 mg of free base correspond to 72 mg of drug substance. | | | | |

### Solubility tests

Solubility of IP2015 was determined in both aqueous and non-aqueous vehicles. In non-aqueous liquids, the solubility was visually confirmed in progressive dilutions. The solubility of IP2015 in pH specific aqueous solutions was evaluated, the solubility was visually confirmed, and the content was evaluated by HPLC.

**Table 2. Solubility of IP2015 in different media.**

| **Vehicle** | **Solubility (mg/ml)** | **Inference (Ph.Eur)** |
|---|---|---|
| Ethanol | <1 | Very slightly soluble |
| N,N-dimethyl acetamide | <1 | Very slightly soluble |
| Dimethyl sulfoxide | 5 to 10 | Slightly soluble |
| N-methyl-2-pyrrolidone | <1 | Very slightly soluble |
| Propylene glycol | 1 to 5 | Slightly soluble |
| Polyethylene glycol 400 | <1 | Very slightly soluble |
| Glycerine | <1 | Very slightly soluble |
| pH 1.2 HCl (0.1N) | 0.017 | Very slightly soluble |
| pH 3.0 Citrate buffer | 0.839 | Very slightly soluble |
| pH 4.0 Citrate buffer | 0.849 | Very slightly soluble |
| pH 3.0 Acetate buffer | 1.173 | Slightly soluble |
| pH 4.5 Acetate buffer | 0.981 | Very slightly soluble |
| pH 5.8 Potassium Phosphate buffer | 0.104 | Very slightly soluble |
| pH 6.8 Potassium Phosphate buffer | 0.146 | Very slightly soluble |
| pH 7.5 Potassium Phosphate buffer | 0.084 | Very slightly soluble |
| Purified Water | 0.738 | Very slightly soluble |

### Results

As seen from table 2, IP2015 has solubility lower than 1 mg/mL in aqueous solutions across physiological pH. It was unexpectedly found that the presence of the binder in the solution allowed for a homogeneous and consistent distribution of IP2015 in the drug layer. In contrast, when a solubility enhancer was used to improve the solubility of IP2015, such as hydroxypropyl 2-beta cyclodextrin (HPβCD), the concentration of the compound was not enough to form a good drug layer.

### Example 2: IP2015 release in solution

### Aim

To study the release of IP2015 for compositions according to the present disclosure.

### Materials and Methods

The dissolution profile of IP2015 IR pellets (part finished IMP) and IP2015 IR capsules is determined using 0.1N HCl Buffer.

### Dissolution conditions:

| **Parameter** | **Condition** |
|---|---|
| Media | 0.1N HCl buffer |
| Volume | 900 mL |
| Apparatus | USP-I (Basket) |
| RPM | 50 |
| Temperature | 37.0° + 0.5°C |
| Sampling volume | 10 mL |
| Time points | 5, 10, 15, 30 min |

Dissolution samples are being analyzed with a gradient reverse phase HPLC method, The mobile phase is buffer solution (0.1% orthophosphoric acid and water): acetonitrile (85:15). The detection is by DAD.

To study the effect of sodium sulphite, two different samples of pellets were prepared according to example 1, with and without sodium sulphite. The dissolution rate of samples corresponding to 5 mg of IP2015 from the pellets without encapsulation was studied.

To study the effect of encapsulation, the dissolution rate of IP2015 from both encapsulated and non-encapsulated pellets was assessed using samples corresponding to 5 mg of IP2015, prepared according to example 1. For comparison, dissolution of non-encapsulated samples corresponding to 10 mg of IP2015 was also assessed.

### Results

The dissolution profile of samples with and without sodium sulphite is shown in Table 3.

**Table 3. Drug release from 5 mg IP2015 IR pellet samples with and without sodium sulphite.**

| **Time** / **min** | **Drug release (%)** | |
|---|---|---|
| | **with Na₂SO₃** | **without Na₂SO₃** |
| 5 | 96 | 103 |
| 10 | 97 | 107 |
| 15 | 98 | 108 |
| 20 | 99 | 108 |
| 30 | 99 | 108 |
| 45 | 99 | 108 |
| 60 | 99 | 108 |

Impact of sodium sulphite on the dissolution profile was observed. The dissolution profiles of scale-up batches, which had sodium sulphite in the drug layering coating, showed a faster dissolution profile compared to a scale-up batch, which does not have sodium sulphite in the drug layer.

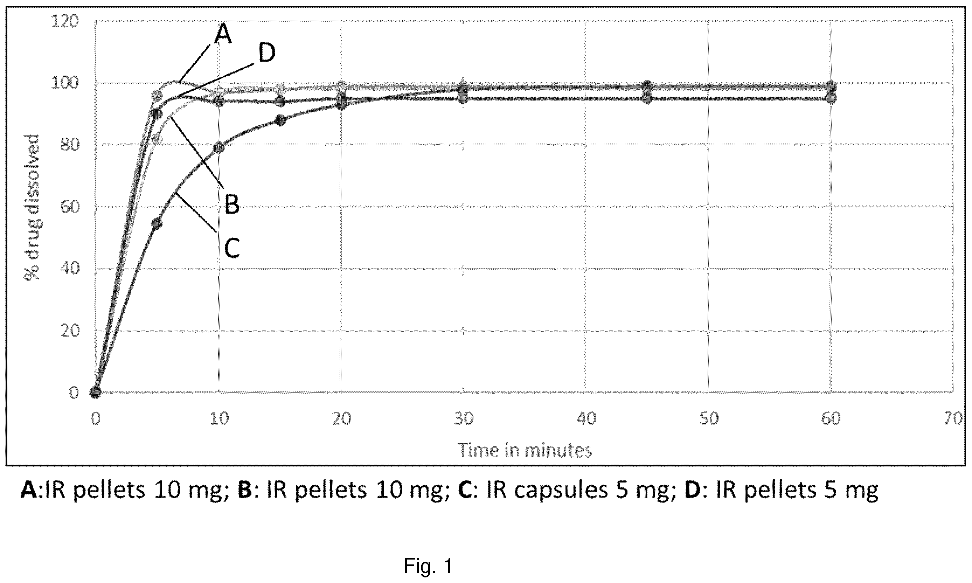

Figure 1 shows the release profile of IP2015 IR pellets and capsules . The dissolution profile is as expected. The IP2015 IR capsule fulfil the requirements for an immediate release oral dosage form with 80% released after 10 min and > 90% released after 20 min.

### Conclusion

It was surprisingly found that the presence of sodium sulphite enhances the dissolution rate from the IR compositions.

### Example 3: In vivo pharmacokinetics of IP2015 IR composition

### Aim

To study the pharmacokinetic parameters of the compositions according to the present disclosure.

### Materials and methods

The pharmacokinetic profiles of different formulations of IR Pellets and one solution formulation (DiB) of IP2015 as reference item were investigated in Göttingen minipigs, after single oral administration.

Animals were fasted prior to dosing. Each formulation was administered to the animals by a single administration; a wash-out period of 7 days was allowed after each day of dosing. A single dose of 0.5mg/kg was administered orally, by capsule, with a washout period of 7 days after each day of dosing. A single dose (0.25mL/kg) of reference item solution was administered orally, by gavage.

Assessment of mortality was conducted twice each day. All clinical signs were recorded at baseline and at the same time daily during the study (approximately 1-1.5, 2-2.5 and 3-3.5 hours after dosing). Fasted body weight was recorded on the day of allocation and the day before dosing.

On each day of dosing, blood samples were collected from the jugular vein (other veins were used, if necessary) at approximately the following time points: pre-dose, 0.5, 1, 2, 3, 4, 6, 8, 10, 12, 24, 36 and 48 hours after the first administration.

At each sampling time, at least 1.0 mL of blood was collected and transferred into tubes containing K₂EDTA anticoagulant and, centrifuged at room temperature, divided in two aliquots: [Aliquot A] of approximately 250 µL and the second [Aliquot B] with the plasma remaining) and frozen at -70 ± 10°C.

The concentration of IP2015 in minipig plasma was determined via LC-MS/MS method by The Bioanalytical Department, Syngene International Ltd. During analysis, standard and quality control samples were distributed throughout each batch of study samples analysed. Plasma concentration data was used for toxicokinetic evaluation.

PK parameters were determined from the individual minipig plasma concentration versus time data by a non-compartmental analysis with uniform weighting using Phoenix^{®} WinNonlin^{®} validated version 8.2. The PK parameters that were calculated includes the area under the plasma concentration-time curve until last quantifiable concentration (AUClast), AUCall, peak plasma concentration (Cₘₐₓ), time to reach peak plasma concentration (Tmax), Cₗₐₛₜ, Tₗₐₛₜ, AUC_{inf} and terminal elimination half-life (t1/2). Plasma concentrations and TK parameters are presented in ng unit.

### Results

The results are displayed in Table 4 and Table 5.

**Table 4. Mean pharmacokinetic parameters of IP2015 in different formulations.**

| | **IR** | **DiB** |
|---|---|---|
| **Dose** (mg/kg) | 0.5 | 0.5 |
| **Cₘₐₓ** (ng/mL) | 17.026 | 25.135 |
| **Tₘₐₓ** (h) | 3.33 | 2.5 |
| **AUC₀₋₄₈ₕ** (h*ng/mL) | 336.317 | 399.6 |
| **Cₗₐₛₜ** | 2.226 | 2.416 |
| **Tₗₐₛₜ** | 48 | 48 |
| **Half-life** (h) | 13.58 | 15.03 |
| **AUCₗₐₛₜ** (h*ng/mL) | 336.317 | 399.6 |
| **AUC_{INF_obs}** (h*ng/mL) | 390.254 | 459.579 |

**Table 5. Systemic exposure ratios IR vs. DiB.**

| | **Ratio (IR** / **DiB)** |
|---|---|
| **Cₘₐₓ** | 0.68 |
| **AUC₀₋₄₈ₕ** | 0.84 |
| **Tₘₐₓ** | 1.3 |

### Conclusion

It was observed that the compositions according to the present disclosure produces a slight increase in Tₘₐₓ, and a decrease in Cₘₐₓ but the exposure of IP2015 is maintained.

### Example 4. Clinical trial studying pharmacokinetics of IP2015 formulations in human.

### Aim

To study the pharmacokinetics of immediate release (IR) compositions of IP2015 according to the present disclosure in humans.

### Materials and Methods

The study is a 3-way crossover study in which pharmacokinetic parameters of IP2015 immediate release compositions according to the present disclosure were studied after oral administration. For comparison, the results were compared to a solution of IP2015. The compositions were prepared as described in example 1.

The study was a crossover study performed in 12 healthy subject that received single doses of 5 mg of IP2015 in different formulations with sufficient wash-out in between. After administration of the compositions, the plasma blood levels of IP2015 monitored at different timepoints.

### Results

The results are shown in Table 6 and Table 7.

**Table 6. Pharmacokinetic parameters of IR and DiB compositions.**

| | **IR composition** | | | | **DiB** | | | |
|---|---|---|---|---|---|---|---|---|
| | **N** | **Min** | **Median** | **Max** | **N** | **Min** | **Median** | **Max** |
| T_{lag} (h) | 12 | 0 | 0 | 0 | 12 | 0 | 0 | 0 |
| tₘₐₓ (h) | 12 | 1.97 | 4.98 | 7.95 | 12 | 0.98 | 3.5 | 6.07 |
| Cₘₐₓ (ng/mL) | 12 | 5.39 | 8.81 | 12.5 | 12 | 6.25 | 8.69 | 14.4 |
| AUCₗₐₛₜ (h*ng/mL) | 12 | 182 | 260 | 410 | 12 | 186 | 251 | 402 |
| AUC_{inf} (h*ng/mL) | 10 | 193 | 301 | 479 | 9 | 199 | 284 | 496 |
| AUC₀₋₂₄ (h*ng/mL) | 12 | 98.7 | 146 | 209 | 12 | 106 | 147 | 219 |

**Table 7. Systemic exposure ratios IR vs. DiB**

| | **IR** / **DiB ratio** | | | | |
|---|---|---|---|---|---|
| | **N** | **Min** | **Median** | **Max** | **Geom. mean (CV%)** |
| **Cₘₐₓ** | 12 | 0.84 | 1.03 | 1.23 | 1.011 (11.91 %) |
| **AUCₗₐₛₜ** | 12 | 0.9 | 0.99 | 1.16 | 1.004 (8.05 %) |
| **AUC_{inf}** | 8 | 0.88 | 1 | 1.18 | 1.012 (10.55 %) |

### Conclusion

It was observed that the compositions according to the present disclosure produces a slight increase in tₘₐₓ, and a decrease in Cₘₐₓ but the exposure of IP2015 is maintained. Thus, the composition of the present disclosure provides for a fast release that closely matches the drug-in-bottle formulation.

### Example 5. Clinical trial studying adverse events and efficacy of treatment of erectile dysfunction with IP2015 in humans (for reference)

The study investigated the effects of repeat single oral doses of IP2015 on male subjects with erectile dysfunction (ED) on ability to develop and maintain an erection, as well as safety and tolerability of single oral doses of IP2015, effects on penile rigidity and tumescence during visual stimulation, effects on sperm count and motility, and any possible relationship between plasma levels of IP2015, its efficacy and safety.

### Materials and Methods

### Study design

130 subjects were divided into 3 groups upon which the study was conducted in parallel. Each subject was dosed on four occasions with either 5 mg IP2015, 10 mg IP2015 or matched placebo. In each group, subjects were randomized evenly to each of the three study treatments and received the same treatment at each visit.

The study lasted approximately 8 weeks and consisted of the following:
- A screening visit (up to 21 days prior to the baseline visit)
- A baseline visit (Day -7)
- Outpatient visits on Week 1 (Day 1), Week 2, Week 3 and Week 4
- A follow-up visit a minimum of 7 to 10 days after their final dose.

On Day -7, and at Week 1 (Day 1), Week 2, Week 3, Week 4 and Follow-up visits, all groups were asked to complete the IIEF-15 questionnaire. Group 2 was asked to complete a Visual Stimulation Assessment using a RigiScan Plus Monitor on Day -7, Week 1 (Day 1) and Week 4. Group 2 was also required to provide blood samples for pharmacokinetic assessment on Week 1 (Day 1) and Week 4. Group 3 was required to provide semen samples on Day -7 and Week 4.

### Participants

The patients were otherwise healthy male subjects with ED as determined from an IIEF-5 score of ≤16, with a body mass index of 18 to 35 kg/m² (inclusive), of any ethnic origin. Subjects were aged between 18 to 59 years, inclusive.

### Dose and Mode of Administration

There were three potential study treatments of 5 mg IP2015, 10 mg IP2015 or matched placebo. In each group, subjects were randomized evenly to each of the three study treatments and received the same treatment on each visit. IP2015 or matched placebo was administered once in the morning of Week 1 (Day 1), Week 2, Week 3, and Week 4 as an oral solution in the fasted state. The dose was taken with 240 mL of water at room temperature. Subjects had fasted 2 hours prior to dosing until 4 hours post-dose. Water was allowed ad libitum except for 1 hour before and 1 hour after dosing.

### Evaluation

Efficacy was assessed through the International Index of Erectile Function (IIEF)-15 questionnaire. The change from baseline in responses to questions of the IIEF-15 questionnaire, including questions regarding erectile function, orgasmic function, sexual desire, intercourse satisfaction and overall satisfaction domain scores, was determined at different time points of the study. RigiScan assessments during the stimulus assessment, and sperm count and motility by semen sample collection was used for efficacy evaluation. Safety was assessed through AE reporting, 12-lead ECG, vital signs, physical examinations, and clinical laboratory evaluations. Pharmacokinetics was assessed by blood sampling.

### Results

### Efficacy of IP2015

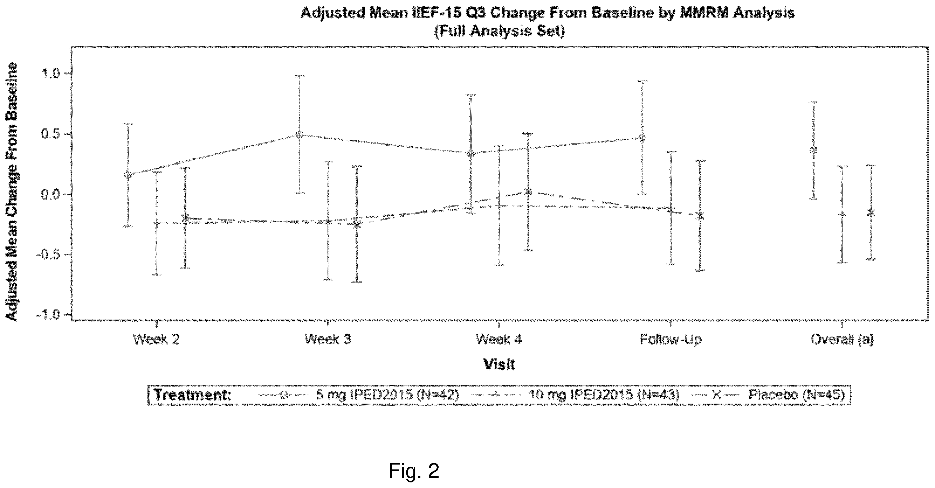

The results for Q3 in the IIEF-15: "When you attempted intercourse, how often were you able to penetrate (enter) your partner during the last week?" are shown in Figure 2. The results for Q3 were significant for treatment with 5 mg IP2015 at week 3 versus placebo (p=0.034) and versus baseline (p=0.046). In the overall score for Q3, there was also a tendency to difference versus placebo (p=0.07) and versus baseline (p=0.07).

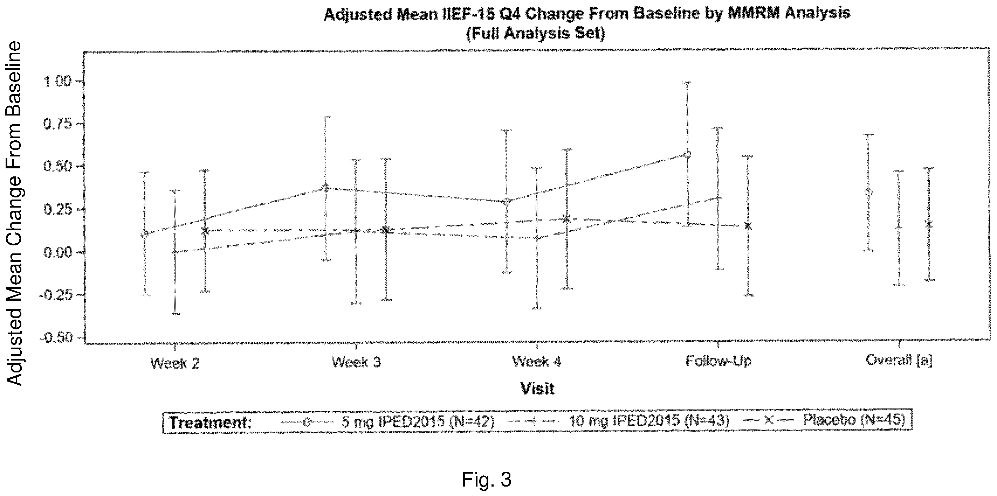

The results for Q4 in the IIEF-15: "During sexual intercourse, how often were you able to maintain your erection after you had penetrated (entered) your partner?" are shown in Figure 3. The results for Q4 show that there was a tendency to difference versus baseline (p=0.056) for the 5 mg in the overall score, though there was no difference compared to placebo (p=0.44).

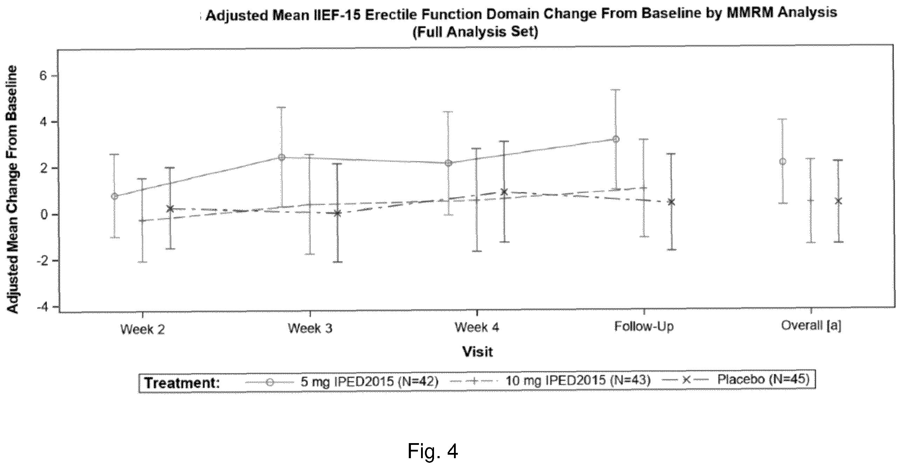

The overall response to questions of IIEF-15 is shown in Figure 4, the results were significant for treatment with 5 mg IP2015 versus baseline at follow-up (p= 0.0046) and tendency versus placebo (p=0.07) and the same was the case for the overall score for 5 mg versus baseline (p= 0.0032) and tendency versus placebo (p=0.10).

In figures 2, 3 and 4, the results show the change from baseline for patients treated with four doses of placebo (n=45), 5 mg (n=42), and 10 mg (n=43) IP2015 dosed in parallel at days 1, 8, 15, and 22. The results are means±SE. The statistical differences were performed with Mixed Model Repeated Measures (MMRM).

### Adverse events

Treatment-emergent adverse effects for the low-dose (5 mg) IP2015 were comparable to the placebo group. The TEAEs were dose-dependent, mild and moderate and slightly increased in the high dose compared to the low dose of IP2015. No severe side effects were observed at any of the doses of IP2015 (Table 8).

The results for semen analysis were complete and the treatment did not have negative effects on sperm count, motility, or morphology.

**Table 8. Treatment-emergent adverse events**

| TEAE | IP2015 5mg, N=42 | IP2015 10 mg, N=43 | Placebo, N=45 |
|---|---|---|---|
| Any TEAE | 32 (76.2 %), 61 | 34 (79.1 %), 141 | 31 (68.9 %), 63 |
| Related* | 10 (23.8 %), 19 | 23 (53.5 %), 84 | 8 (17.8 %), 11 |
| Mild | 29 (69.0 %), 56 | 33 (76.7%), 132 | 27 (60.0%), 57 |
| Moderate | 3 (7.1%), 5 | 7 (16.3%), 9 | 6 (13.3%), 6 |
| Severe | 0 | 0 | 0 |
| Headache | 11 (26.2 %), 15 | 16 (37.2 %), 27 | 14 (31.1 %), 21 |
| Dizziness | 3 (7.1 %), 3 | 12 (27.9 %), 15 | 2 (4.4 %), 2 |
| Nausea | 1 (2.4 %), 1 | 8 (18.6 %), 15 | 1 (2.2 %), 1 |

### Conclusion

Overall, these results, show that 5 mg doses that IP2015 are effective in treating erectile dysfunction and are safely tolerated. There was a significant effect of the 5 mg dose of IP2015 (pudafensine) on the Q3 in the IIEF-15 score compared to baseline and placebo. There were tendencies regarding the overall IIEF-15 score showing a clinically significant score change of 2, which is significant for a low dose of IP2015 versus baseline and p=0.10 versus placebo.

Treatment-emergent adverse effects for the 5 mg dose of IP2015 were comparable to the placebo group. The TEAEs were dose-dependent, only mild and moderate effects were observed. No severe TEAEs were present.

The higher efficacy and lower incidence of TEAEs at 5 mg of IP2015 compared to 10 mg demonstrates the need for the present solid dosage formulation that mimic the pharmacokinetics of the drug-in-bottle (DiB) used in this study without compromising exposure.

### References

Sandeep Kalepu et al. Insoluble drug delivery strategies: review of recent advances and business prospects. Acta pharmaceutica Sinica B, vol. 5, no. 5, 1 September 2015, p. 442-453. DOI: 10.1016/j.apsb.2015.07.003

## Claims

1. A composition comprising pellets, said pellets comprising or consisting of:
a. a pellet core; and
b. a drug layer covering the core, the drug layer comprising a compound of formula (I) 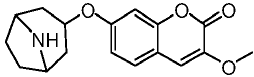 formula (I), or a pharmaceutically acceptable salt thereof.

2. The composition according to claim 1, wherein the drug layer further comprises a binder selected from cellulose derivatives, such as hydroxypropylmethyl cellulose (HPMC), or hydroxypropyl cellulose (HPC); gelatin; polyalkylene glycols, such as PEO; and polyvinyl pyrrolidone (PVP).

3. The composition according to claim 2, wherein the drug layer comprises the binder in an amount from 0.5 to 10% by weight of the pellets.

4. The composition according to any one of claims 2-3, wherein the binder is HPMC.

5. The composition according to any one of the preceding claims, wherein the drug layer comprises HPMC in an amount from 1% to 5% by weight of the pellets.

6. The composition according to any one of the preceding claims, wherein the pellet core comprises or consists of spheres made of a material selected form: microcrystalline cellulose (MCC); sugars, such as sucrose, xylitol, mannitol, and lactose; starch, silica, tartaric acid and calcium carbonate, preferably microcrystalline cellulose (MCC).

7. The composition according to any one of the preceding claims, wherein the drug layer comprises the compound of formula (I), or a pharmaceutically acceptable salt thereof, in an amount of 0.5% to 5% by weight of the pellets.

8. The composition according to any one of the preceding claims, wherein the compound is exo-7-[(8-azabicyclo[3.2.1]octan-3-yl)oxy]-3-methoxy-chromen-2-one or a pharmaceutically acceptable salt thereof, such as a hydrochloride salt.

9. The composition according to any one of the preceding claims, wherein said composition is an immediate release composition of an active ingredient consisting of a compound of formula (I), 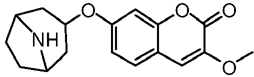 formula (I), or a pharmaceutically acceptable salt thereof, the composition comprising pellets, said pellets comprising or consisting of:
a) an inert pellet core comprising microcrystalline cellulose (MCC);
b) a drug layer comprising: the compound of formula (I), or a pharmaceutically acceptable salt thereof, in an amount of 0.5% to 5% by weight of the pellets, and HPMC in an amount of 0.5% to 5% by weight of the pellets, wherein the drug layer covers the inert core.

10. The composition according to any one of the preceding claims , wherein the drug layer further comprises additional excipients selected from: pH modifiers and/or stabilizers.

11. The composition according to claim 10, wherein the stabilizer is sodium sulphite.

12. The composition according to any one of claims 10-11, wherein the drug layer comprises sodium sulphite in an amount from 0.05 % to 1% by weight of the pellets.

13. The composition according to any one of the preceding claims, wherein the composition comprises an amount of the compound of formula (I) of 0.5 mg to 10 mg, preferably wherein the composition comprises 5 mg of the compound of formula (I).

14. The composition according to any one of the preceding claims, wherein the composition is in the form of a pharmaceutical dosage form or a unit dosage form, such as a capsule.

15. The composition according to any one of the preceding claims, wherein the composition is for oral administration.

## Patentansprüche

1. Zusammensetzung, die Pellets umfasst, wobei die Pellets Folgendes umfassen oder daraus bestehen:
a. einen Pelletkern; und
b. eine Wirkstoffschicht, die den Kern bedeckt, wobei die Wirkstoffschicht eine Verbindung der Formel (I) 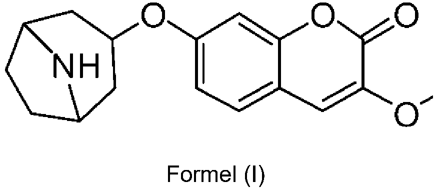 oder ein pharmazeutisch verträgliches Salz davon umfasst.

2. Zusammensetzung nach Anspruch 1, wobei die Wirkstoffschicht ferner ein Bindemittel umfasst, das ausgewählt ist aus Cellulosederivaten, wie Hydroxypropylmethylcellulose (HPMC) oder Hydroxypropylcellulose (HPC); Gelatine; Polyalkylenglykolen, wie PEO; und Polyvinylpyrrolidon (PVP).

3. Zusammensetzung nach Anspruch 2, wobei die Wirkstoffschicht das Bindemittel in einer Menge von 0,5 bis 10 Gew.-% der Pellets umfasst.

4. Zusammensetzung nach einem der Ansprüche 2 bis 3, wobei es sich bei dem Bindemittel um HPMC handelt.

5. Zusammensetzung nach einem der vorhergehenden Ansprüche, wobei die Wirkstoffschicht HPMC in einer Menge von 1 bis 5 Gew.-% der Pellets umfasst.

6. Zusammensetzung nach einem der vorhergehenden Ansprüche, wobei der Pelletkern Kugeln umfasst oder aus Kugeln besteht, die aus einem Material hergestellt sind, das ausgewählt ist aus: mikrokristalliner Cellulose (MCC); Zuckern, wie Saccharose, Xylitol, Mannitol und Lactose; Stärke, Siliciumdioxid, Weinsäure und Calciumcarbonat, vorzugsweise mikrokristalliner Cellulose (MCC).

7. Zusammensetzung nach einem der vorhergehenden Ansprüche, wobei die Wirkstoffschicht die Verbindung der Formel (I) oder ein pharmazeutisch verträgliches Salz davon in einer Menge von 0,5 bis 5 Gew.-% der Pellets umfasst.

8. Zusammensetzung nach einem der vorhergehenden Ansprüche, wobei es sich bei der Verbindung um exo-7-[(8-Azabicyclo[3.2.1]octan-3-yl)oxy]-3-methoxy-chromen-2-on oder ein pharmazeutisch verträgliches Salz davon, wie ein Hydrochloridsalz, handelt.

9. Zusammensetzung nach einem der vorhergehenden Ansprüche, wobei es sich bei der Zusammensetzung um eine Zusammensetzung mit sofortiger Freisetzung eines Wirkstoffs handelt, der aus einer Verbindung der Formel (I), 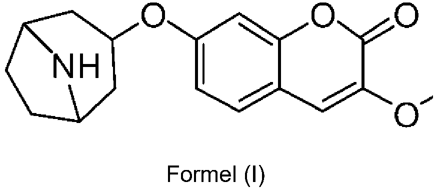 oder einem pharmazeutisch verträglichen Salz davon besteht, wobei die Zusammensetzung Pellets umfasst, wobei die Pellets Folgendes umfassen oder daraus bestehen:
a) einen inerten Pelletkern, der mikrokristalline Cellulose (MCC) umfasst;
b) eine Wirkstoffschicht, die Folgendes umfasst: die Verbindung der Formel (I) oder ein pharmazeutisch verträgliches Salz davon in einer Menge von 0,5 bis 5 Gew.-% der Pellets und HPMC in einer Menge von 0,5 bis 5 Gew.-% der Pellets, wobei die Wirkstoffschicht den inerten Kern bedeckt.

10. Zusammensetzung nach einem der vorhergehenden Ansprüche, wobei die Wirkstoffschicht ferner zusätzliche Hilfsstoffe umfasst, die ausgewählt sind aus: pH-Modifiziermitteln und/oder Stabilisatoren.

11. Zusammensetzung nach Anspruch 10, wobei es sich bei dem Stabilisator um Natriumsulfit handelt.

12. Zusammensetzung nach einem der Ansprüche 10 bis 11, wobei die Wirkstoffschicht Natriumsulfit in einer Menge von 0,05 bis 1 Gew.-% der Pellets umfasst.

13. Zusammensetzung nach einem der vorhergehenden Ansprüche, wobei die Zusammensetzung eine Menge der Verbindung der Formel (I) von 0,5 mg bis 10 mg umfasst, vorzugsweise wobei die Zusammensetzung 5 mg der Verbindung der Formel (I) umfasst.

14. Zusammensetzung nach einem der vorhergehenden Ansprüche, wobei die Zusammensetzung in Form einer pharmazeutischen Dosierungsform oder einer Einheitsdosierungsform, wie einer Kapsel, vorliegt.

15. Zusammensetzung nach einem der vorhergehenden Ansprüche, wobei die Zusammensetzung zur oralen Verabreichung bestimmt ist.

## Revendications

1. Composition comprenant des granulés, lesdits granulés comprenant ou étant constitués de :
a. un noyau de granulé ; et
b. une couche de médicament recouvrant le noyau, la couche de médicament comprenant un composé de formule (I) 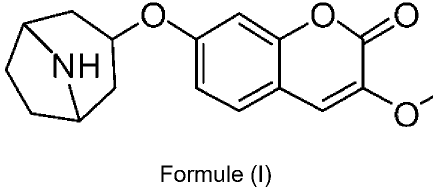 ou un sel pharmaceutiquement acceptable de celui-ci.

2. Composition selon la revendication 1, dans laquelle la couche de médicament comprend en outre un liant sélectionné parmi les dérivés de cellulose, tels que l'hydroxypropylméthylcellulose (HPMC) ou l'hydroxypropylcellulose (HPC) ; la gélatine ; les polyalkylèneglycols, tels que le PEO ; et la polyvinylpyrrolidone (PVP).

3. Composition selon la revendication 2, dans laquelle la couche de médicament comprend le liant en une quantité de 0,5 à 10 % en poids des granulés.

4. Composition selon l'une quelconque des revendications 2 à 3, dans laquelle le liant est la HPMC.

5. Composition selon l'une quelconque des revendications précédentes, dans laquelle la couche de médicament comprend la HPMC en une quantité de 1 % à 5 % en poids des granulés.

6. Composition selon l'une quelconque des revendications précédentes, dans laquelle le noyau de granulé comprend ou est constitué de sphères constituées d'un matériau sélectionné parmi : la cellulose microcristalline (MCC) ; les sucres, tels que le saccharose, le xylitol, le mannitol et le lactose ; l'amidon, la silice, l'acide tartrique et le carbonate de calcium, de préférence la cellulose microcristalline (MCC).

7. Composition selon l'une quelconque des revendications précédentes, dans laquelle la couche de médicament comprend le composé de formule (I), ou un sel pharmaceutiquement acceptable de celui-ci, en une quantité de 0,5 % à 5 % en poids des granulés.

8. Composition selon l'une quelconque des revendications précédentes, dans laquelle le composé est la exo-7-[(8-azabicyclo[3.2.1]octan-3-yl)oxy]-3-méthoxy-chromén-2-one ou un sel pharmaceutiquement acceptable de celui-ci, tel qu'un sel de chlorhydrate.

9. Composition selon l'une quelconque des revendications précédentes, dans laquelle ladite composition est une composition à libération immédiate d'un principe actif constitué d'un composé de formule (I), 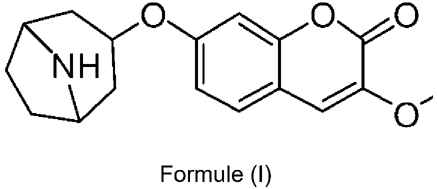 ou d'un sel pharmaceutiquement acceptable de celui-ci, la composition comprenant des granulés, lesdits granulés comprenant ou étant constitués de :
a) un noyau de granulé inerte comprenant de la cellulose microcristalline (MCC) ;
b) une couche de médicament comprenant : le composé de formule (I), ou un sel pharmaceutiquement acceptable de celui-ci, en une quantité de 0,5 % à 5 % en poids des granulés, et de la HPMC en une quantité de 0,5 % à 5 % en poids des granulés, dans laquelle la couche de médicament recouvre le noyau inerte.

10. Composition selon l'une quelconque des revendications précédentes, dans laquelle la couche de médicament comprend en outre des excipients supplémentaires sélectionnés parmi : des modificateurs de pH et/ou des stabilisants.

11. Composition selon la revendication 10, dans laquelle le stabilisant est le sulfite de sodium.

12. Composition selon l'une quelconque des revendications 10 à 11, dans laquelle la couche de médicament comprend du sulfite de sodium en une quantité de 0,05 % à 1 % en poids des granulés.

13. Composition selon l'une quelconque des revendications précédentes, dans laquelle la composition comprend une quantité du composé de formule (I) de 0,5 mg à 10 mg, de préférence dans laquelle la composition comprend 5 mg du composé de formule (I).

14. Composition selon l'une quelconque des revendications précédentes, dans laquelle la composition se présente sous la forme d'une forme posologique pharmaceutique ou d'une forme posologique unitaire, telle qu'une capsule.

15. Composition selon l'une quelconque des revendications précédentes, dans laquelle la composition est destinée à une administration orale.
